(19) Europäisches Patentamt
European Patent Office
Office européen des brevets

(11) EP 1 645 277 A1

(12) **EUROPEAN PATENT APPLICATION**
published in accordance with Art. 158(3) EPC

(43) Date of publication:
**12.04.2006 Bulletin 2006/15**

(21) Application number: **04738334.4**

(22) Date of filing: **02.07.2004**

(51) Int Cl.:
***A61K 31/529*** *(2000.01)* ***A61K 9/19*** *(1995.01)*
***A61P 25/04*** *(2000.01)*

(86) International application number:
**PCT/CN2004/000736**

(87) International publication number:
**WO 2005/004874 (20.01.2005 Gazette 2005/03)**

(84) Designated Contracting States:
**AT BE BG CH CY CZ DE DK EE ES FI FR GB GR HU IE IT LI LU MC NL PL PT RO SE SI SK TR**
Designated Extension States:
**LT LV**

(30) Priority: **14.07.2003 CN 03146020**

(71) Applicant: **NANNING MAPLE LEAF PHARMACEUTICAL CO., LTD.**
**530003 Nanning, Guangxi (CN)**

(72) Inventors:
- **ZHANG, Xiao**
  **530003 Nanning, Guangxi (CN)**
- **KANG, Yuhong**
  **530003 Nanning, Guangxi (CN)**
- **HUANG, Xiaoyan**
  **530003 Nanning, Guangxi (CN)**

(74) Representative: **Vossius & Partner**
**Siebertstrasse 4**
**81675 München (DE)**

# (54) STABLE TETRODOTOXIN FREEZE DRYING MEDICINAL PREPARATION

(57) The present invention provides a freeze-dried pharmaceutical formulation comprising tetrodotoxin or an analog thereof in an amount of 0.5-60 μg per dose, which has good stability and is safe for use by injection in humans and can be stored at room temperature for a long time. Said formulation also contains compounds, such as compounds containing glucosidic bonds selected from any one of disaccharides, ficolls, the derivatives thereof or their mixtures capable of reducing C-4 hydroxyl activity of the tetrodotoxin molecule or the analogs thereof, and a co-solvent(s) which improves solubility of tetrodotoxin or the analogs thereof.

EP 1 645 277 A1

## Description

### FIELD OF THE INVENTION

This invention relates to a freeze-dried pharmaceutical formulation, particularly, a freeze-dried pharmaceutical formulation of tetrodotoxin safe for use by injection in humans.

### BACKGROUND OF THE INVENTION

Being a naturally-occurring non-protein nerve toxin, tetrodotoxin binds with the SS1/SS2 subunit of sodium channels with high specificity and high affinity, and has been widely used as a tool drug in pharmacological research, particularly neuropharmacology and muscular physiology, for decades. On the market, Sigma-Aldrich supplies a typical tetrodotoxin product, a freeze-dried solid citrate powder containing 1 mg of tetrodotoxin (product number T5881).

In addition to its use in scientific research, therapeutic applications were discovered by the company of the inventors, which as early as 1998 applied for a Class One new drug approval for its tetrodotoxin injection (aqueous solution) for treatment of drug addiction and pain (U.S. Patent Nos. US 5,846,975, Pan et al.; and US 6,407,088, Dong et al.).

However, the injectable tetrodotoxin solution (aqueous solution) is very sensitive to temperature. Tetrodotoxin readily degrades under higher temperatures, i.e., the higher the temperature is, the faster it degrades. Once the content of tetrodotoxin, the active pharmaceutical ingredient, is reduced to less than 90% of the labeled amount, or the relative content of related substances exceeds the specified limit by medical standards (greater than the main peak area of a control solution), the drug will not be suitable for clinical use anymore. The inventors examined the tetrodotoxin injection for the content of tetrodotoxin and the relative content of related substances by HPLC, and discovered that the content of tetrodotoxin is changed with the temperature and the duration of storage. The results suggest that the content of tetrodotoxin declined to 91.9% (reduced by 8.1%) on day 1 and further to 89.37% on day 3 at 40°C, a total drop of 10.63% (See Table 1). These results show that the tetrodotoxin injection will fail to meet the medical criteria as its content will decline to less than the specified limit after being exposed to 40°C for three days. Moreover, the content of tetrodotoxin declined to 95.34% after the injection had been stored for one month at 25°C, a drop of 4.66%, whereas related substances had a relative content greater than the main peak area of the control solution, exceeding the specified limit and not meeting the medical criteria. After three months, the content of tetrodotoxin declined to 89.77%, a drop of 10.23%, while related substances had a relative content greater than the main peak of the control solution, thus neither met the medical criteria (See Table 2).

These results indicate that the quality of the injectable tetrodotoxin is not maintainable at 25°C, and the content of related substances exceeds the specified limit after one month, not meeting the medical criteria. To ensure the quality and prevent the content of tetrodotoxin from declining and the content of related substances from escalating, the tetrodotoxin injection must be stored in a refrigerator at a temperature range of 4-8°C. This requirement makes its clinical use difficult and inconvenient as the temperature must be kept at 4-8°C at all relevant times including storage, transportation, loading and unloading, wholesale and retail, hospital and administration, otherwise higher temperature can be detrimental to the clinical effectiveness. Therefore, it is necessary to solve this problem by developing a safe and stable product which can be stored at room temperature.

Table 1 Stability Study on Tetrodotoxin Injection (990120A) at 40°C

| Conditions | Duration of Storage | Appearance | Content (%) | Remarks |
|---|---|---|---|---|
| 40°C | Day 0 | Colorless, clear liquid | 100 | Content at day 0 is set to be 100%. |
| | Day 1 | Colorless, clear liquid | 91.9 | |
| | Day 3 | Colorless, clear liquid | 89.37 | |
| | Day 5 | Colorless, clear liquid | 87.45 | |
| | Day 10 | Colorless, clear liquid | 88.06 | |
| Note: The criteria are not met when the content of tetrodotoxin is less than 90%. | | | | |

Table 2 Stability Study on Tetrodotoxin Injection at 25°C

| Duration of Storage (months) | Content (%) | Related substances (%) | Remarks |
|---|---|---|---|
| 0 | 100 | < main peak area of control solution | Content at day 0 is |

Table continued

| Duration of Storage (months) | Content (%) | Related substances (%) | Remarks |
|---|---|---|---|
| 1 | 95.34 | > main peak area of control solution | set to be 100%. |
| 2 | 93.72 | > main peak area of control solution | |
| 3 | 89.77 | > main peak area of control solution | |
| 6 | 82.47 | > main peak area of control solution | |
| Note: The criteria are not met if content of tetrodotoxin is less than 90% or related substances content is greater than the main peak of the control solution. | | | |

The study shows that the tetrodotoxin injection (liquid form) is not stable at room temperature, entailing storage at low temperature at 4-8°C, thus making it inconvenient to transport and store. A simple solution for this problem appears to be a freeze-dried formulation of tetrodotoxin. Generally, a bioactive substance unstable in aqueous solution can have its storage life prolonged by way of freeze drying and dehydrating. Subsequently, the drug can be regenerated by adding sterile water for injection before clinical use. However, the dose of tetrodotoxin for pharmaceutical use is in the range of 0.5~60 μg, which is so low that a solid residue cannot be generated from a solution of tetrodotoxin after freeze drying and dehydrating. Therefore, it is necessary to add a pharmaceutically acceptable excipient(s) so as to provide a framework for a trace amount of tetrodotoxin to attach to, and enabling generation of a solid residue after freeze drying and dehydrating.

However, neither citrate used in the scientific tool drug of tetrodotoxin, nor mannitol, the most popular excipient, is able to generate acceptable results in our experiments. The study shows that, with citrate used as excipient, the appearance of the freeze-dried formulation shrank and disfigured at 40°C, not meeting the medical criteria, while the content of tetrodotoxin declined gradually, and the content of related substances became greater than the main peak of the control solution on day 5, exceeding the specified limit and not meeting the medical criteria either. With mannitol used as excipient, the appearance of the product met the criteria but the content of tetrodotoxin declined gradually, while the content of related substances became greater than the main peak of the control solution on day 5, exceeding the specified limit and not meeting the medical criteria (See Table 3). Storing at 25°C for six months with citrate used as excipient, the content of tetrodotoxin declined to 95.1% from 100% at month zero, a drop of 4.9%. Meanwhile, related substances had a relative content greater than the main peak of the control solution, exceeding the specified limit and not meeting the criteria. With mannitol used as excipient, the content of tetrodotoxin declined to 96.59% at month 6 from 100% of month 0, a drop of 3.41%. Meanwhile, related substances had a relative content greater than the main peak of the control solution, exceeding the specified limit and not meeting the criteria (See Table 4).

Table 3

| Formulation | Citrate | | Mannitol | |
|---|---|---|---|---|
| Appearance | Slack white cake-shaped solid. Nearly shrank and disfigured completely after storage for a day at 40°C. The appearance did not meet the criteria. | | Slack white cake-shaped solid. No obvious change was found after storage for 10 days at 40°C. The appearance met the criteria. | |
| Content and related substances | Tetrodotoxin content (%) | Related substance (%) | Tetrodotoxin Content (%) | Related substance (%) |
| Limit | 90-110 | <main peak area of control solution | 90-110 | < main peak area of control solution |
| Day 0 | 100 | <main peak area of control solution | 100 | < main peak area of control solution |
| Day 1 | 98.97 | <main peak area of control solution | 99.86 | < main peak area of control solution |
| Day 3 | 97.26 | <main peak area of control solution | 99 | <main peak area of control solution |
| Day 5 | 96.19 | >main peak area of control solution | 97.69 | >main peak area of control solution |
| Day 10 | 93.18 | >main peak area of control solution | 95.55 | >main peak area of control solution |

Table 4

| Formulation | Citrate | | Mannitol | |
|---|---|---|---|---|
| Appearance | Slack white cake-shaped solid. Gradually shrank and disfigured into crystal-like substance and attached to the vial walls. Appearance did not meet the criteria. | | Slack white cake-shaped solid. No obvious change was found in storage at room temperature. The appearance met the criteria. | |
| Content and related substances | Tetrodotoxin content (%) | Related substance (%) | Tetrodotoxin content (%) | Related substance (%) |
| Limit | 90-110 | <main peak area of control solution | 90-110 | <main peak area of control solution |
| Month 0 | 100 | <main peak area of control solution | 100 | <main peak area of control solution |
| Month 1 | 98.8 | <main peak area of control solution | 99.69 | <main peak area of control solution |
| Month 2 | 97.2 | <main peak area of control solution | 98.14 | <main peak area of control solution |
| Month 3 | 96.6 | <main peak area of control solution | 98.04 | <main peak area of control solution |
| Month 6 | 95.1 | >main peak area of control solution | 96.59 | >main peak area of control solution |
| Note: The criteria are not met if the content of tetrodotoxin is less than 90% or related substances' content is greater than the main peak of control solution. | | | | |

These results suggest that excipients in existing formulation techniques help formulations stabilize physically. A search for publications did not find any related additives which were capable of improving the chemical stability of a powder formulation containing only a trace amount of tetrodotoxin. The inventors realized that it was necessary to find a new approach to improve the stability of the tetrodotoxin formulation to temperature by working on the chemical structure of tetrodotoxin.

The chemical name of tetrodotoxin is octahydro-12-(hydroxymethyl)-2-imino-5,9:7,10α-dimethano-10αH-[1,3] dioxocino[6,5-d]-pyrimidine-4,7,10,11,12-pentol, with molecular formula $C_{11}H_{17}N_3O_8$ and molecular weight 319.28, which has the following structure:

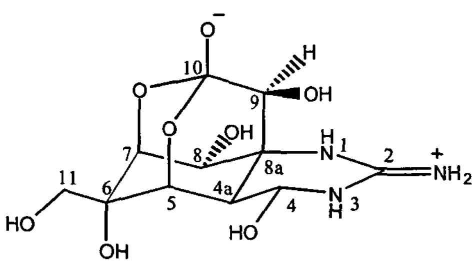

Structure of Tetrodotoxin

Tetrodotoxin darkens above 220°C without decomposition. $[\alpha]_D^{25}$ -8.64 (C=8.55 in diluted acetic acid). pKa 8.76 (water); 9.4 (50% alcohol). It is soluble in diluted acetic acid, and insoluble in water, anhydrous alcohol, and neither soluble in other organic solvents. The toxin is destroyed in strong acids and alkaline solvents (The Merck Index. 13th Ed. 2001, 9318).

Tetrodotoxin in solid state is relatively stable to temperature, but not so in an aqueous solution, particularly at a low concentration in a dilute acid aqueous solution (US 6,559,154, Kang et al.) .

## DETAILED DESCRIPTION OF THE INVENTION

The objective of the present invention provides a freeze-dried pharmaceutical formulation of tetrodotoxin or its analogs.

In order to achieve this objective, the present invention provides a stable freeze-dried pharmaceutical formulation of tetrodotoxin comprising a trace amount of bioactive ingredient, namely, tetrodotoxin or its analogs, stabilizer(s) and co-solvent(s), wherein said tetrodotoxin or its analogs has an amount of 0.5-60 μg per dose, wherein said formulation also contains a compound capable of reducing the activity of C-4 hydroxyl of tetrodotoxin molecule, preferably, a disaccharide or a ficoll, or derivative thereof or one or a mixture of the compounds containing a glucosidic bond.

More specifically, in order to achieve the above goals, the present inventors studied and explored the following:

(1) Why does the tetrodotoxin content decline in an acidic aqueous solution?
(2) What are the degradation products after the tetrodotoxin content declines? What is the difference between the degradation products and tetrodotoxin?
(3) What is the mechanism of degradation of tetrodotoxin?
(4) How can the degradation of tetrodotoxin be prevented?

Question 1: Why does the tetrodotoxin content decline in an acidic aqueous solution?

In 1965, T. Goto et al pointed out that acids catalyze epimerization of tetrodotoxin to form 4-epi-tetrodotoxin and further 4,9-dehydro-tetrodotoxin (Tetrahedron, 1965, Vol.21, 2059-2088). In addition, when heated in reflux, tetrodotoxin was converted into tetrodonic acid (Annals New York Academy of Sciences, 1985, 479:32-43).

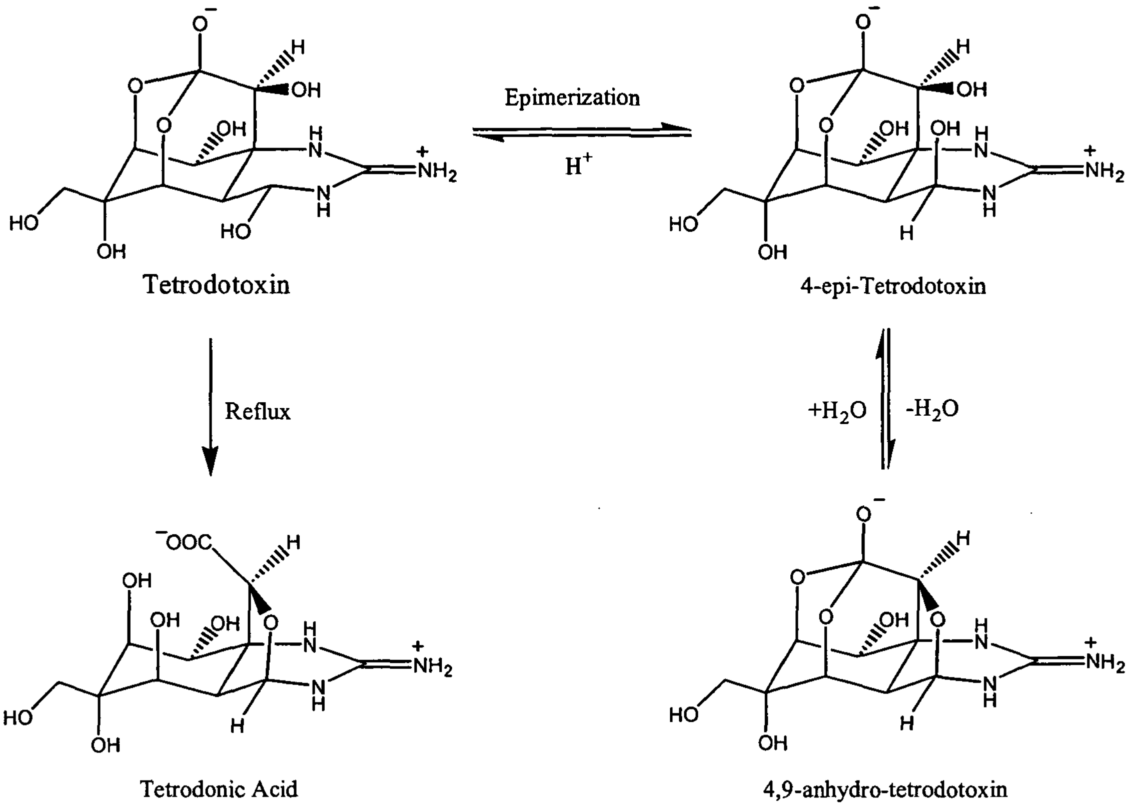

Tetrodotoxin
4-epi-Tetrodotoxin
Tetrodonic Acid
4,9-anhydro-tetrodotoxin

Interconversions between tetrodotoxin and its derivatives

Because tetrodotoxin epimerizes in an acidic aqueous solution into 4-epi-tetrodotoxin and 4,9-dehydro-tetrodotoxin, thus its content declines. Tetrodotoxin turns into tetrodonic acid when heated in reflux.

The aforementioned conclusions were evidenced by the inventors' studies on extraction, purification, and structure modification of tetrodotoxin, as well as on determination of 4-epi-tetrodotoxin and 4,9-dehydro-tetrodotoxin over a long time.

Question 2: What are the degradation products after the tetrodotoxin content declines? What is the difference

between the degradation products and tetrodotoxin?

It is reported that tetrodotoxin turns into 4-epi-tetrodotoxin and 4,9-dehydro-tetrodotoxin in an acidic aqueous solution. To verify this finding, a thermodynamic study was conducted on tetrodotoxin.

The study was carried out as follows: a number of tetrodotoxin solutions having the same concentration but different pH values were equilibrated in aqueous solutions at 80°C±1°C and 90 °C±1°C, respectively. Then samples were taken at various equilibrium time points and frozen to -18°C. The degradation products and their content were measured by HPLC (U.S. Patent No. 6,562,968, Zhou et al.). Results are shown in Tables 5 and 6.

Table 5 Thermodynamic Study on Tetrodotoxin at 80°C (pH 4.67)

| Time (min) | Residue of tetrodotoxin (%) | Degradation products (%) | | | Remarks |
|---|---|---|---|---|---|
| | | 4-epi-tetrodotoxin | 4,9-dehydro-tetrodotoxin | Tetrodonic acid | |
| 0 | 100 | 0 | 0 | 0 | |
| 30 | 89.2 | 4.5 | 4.7 | 0 | |
| 60 | 80.4 | 7.4 | 9.3 | 0 | |
| 90 | 75.2 | 9.1 | 13.5 | 0 | |
| 120 | 70.1 | 9.4 | 17.1 | 0 | |
| 240 | 58.9 | 9.4 | 28.1 | 0 | |

As shown in Table 5, tetrodotoxin turned into 4-epi-tetrodotoxin and 4,9-dehydro-tetrodotoxin in an acidic aqueous solution at 80°C.

Table 6 Thermodynamic Study on Tetrodotoxin at 90°C (pH 4.85)

| Time (min) | Residue of tetrodotoxin (%) | Degradation products (%) | | | Remarks |
|---|---|---|---|---|---|
| | | 4-epi-tetrodotoxin | 4,9-dehydro-tetrodotoxin | Tetrodonic acid | |
| 0 | 98.5 | 0.76 | 0.76 | 0 | |
| 30 | 68.8 | 11.9 | 13.9 | 1.7 | |
| 90 | 53.5 | 9.7 | 29.3 | 4.9 | |
| 120 | 50.6 | 8.8 | 32.7 | 6.1 | |
| 240 | 44 | 7.9 | 34.6 | 9.8 | |

As shown in Table 6, tetrodotoxin turned into 4-epi-tetrodotoxin and 4,9-dehydro-tetrodotoxin in an acidic aqueous solution at 90 °C, and partially further into tetrodonic acid.

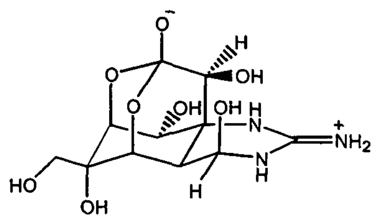
(1)

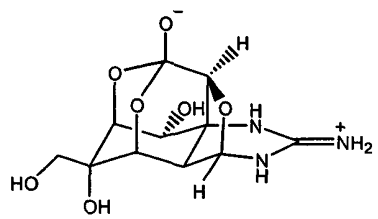
(2)

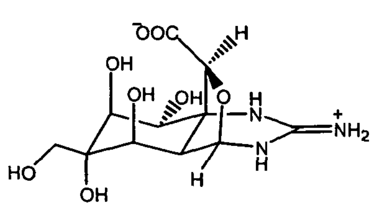
(3)

The common derivatives of Tetrodotoxin
(1) 4-epi-Tetrodotoxin (2) 4,9-anhydro-Tetrodotoxin (3) Tetrodonic acid

The above study confirmed that tetrodotoxin turned into 4-epi-tetrodotoxin and 4,9-dehydro-tetrodotoxin in an acidic aqueous solution at 90 °C, and further into tetrodonic acid at 90 °C.

Tetrodotoxin, 4-epi-tetrodotoxin and 4,9-dehydro-tetrodotoxin have similar chemical properties. 4-Epi-tetrodotoxin has the same molecular formula and molecular weight as tetrodotoxin but its hydroxyl group on C4 has a different stereochemical-position. The molecular formula of 4,9-dehydro-tetrodotoxin has lost an $H_2O$ compared that of tetrodotoxin and 4-epi-tetrodotoxin, and has declined by 18 in molecular weight, but they have significant distinction in bioactivity. For example, the toxicity of tetrodotoxin is 4500 mouse units/milligram; 4-epi-tetrodotoxin, 710 mouse units/milligram; 4,9-dehydro-tetrodotoxin, only 92 mouse units/milligram (Toxicon. 1985, 23:271 ~ 276). Because of such significant differences in bioactivity, tetrodotoxin loses its bioactivity once it is converted into 4-epi-tetrodotoxin or 4,9-dehydro-tetrodotoxin and thus its bioactivity is reduced greatly.

Question 3: What is the chemical mechanism of conversion of tetrodotoxin into 4-epi-tetrodotoxin or 4,9-dehydro-tetrodotoxin?

The chemical structure of tetrodotoxin indicates that C-4 is special as it is adjacent to N, connecting an equatorially positioned hydroxyl and an axially positioned proton. Therefore, the C-4 hydroxyl group of the tetrodotoxin molecule has special chemical and physiological activity. In the presence of $H^+$ in a solution, $H^+$ binds the oxygen atom of the C-4 hydroxyl so that Structure B is inverted from Structure A. Therefore, Structure B loses an $H_2O$ to form Structure C with a cation. In a solution where Structure C binds a water molecule, Structure E or Structure D is formed if the water molecule binds at the position where previous $H_2O$ was lost or at the opposite position to that where the previous $H_2O$ was lost. If $H^+$ is removed from Structure D, Structure A, (i.e., tetrodotoxin) is formed. If $H^+$ is removed from Structure E, Structure F is formed. The difference between Structure F and Structure A is the interchange of positions between the proton and the hydroxyl. The C-4 proton of Structure A is at an axial position, and the hydroxyl is at an equatorial position; while the C-4 proton of Structure F is at an equatorial position and the hydroxyl is at an axial position. Structures like A and F are epimers chemically. The chemical mechanism of epimerization is as follows:

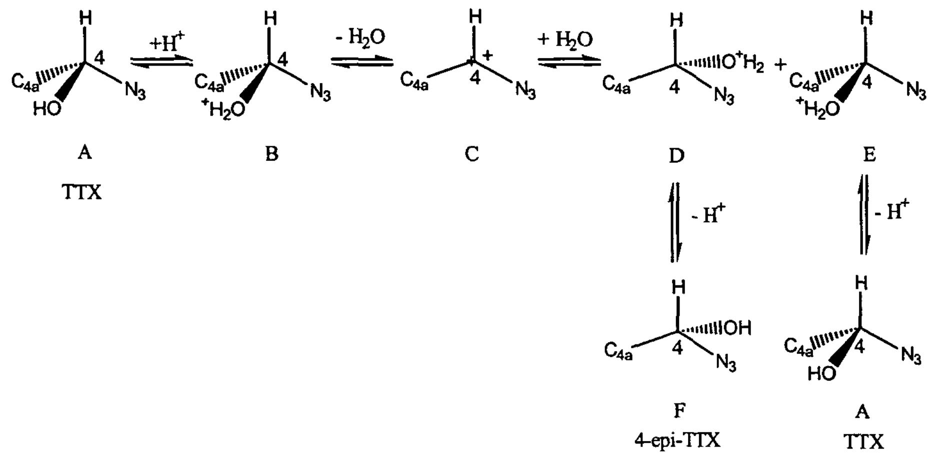


Mechanism of epimerization of the C-4 hydroxyl of group of tetrodotoxin

Structure A is known as "tetrodotoxin", it is the major component of the natural tetrodotoxin ("TTX") extracted from puffer fish. Structure F is known as 4-epi-tetrodotoxin, which is readily converted into more stable 4,9-dehydro-tetrodotoxin because in the presence of $H^+$ an $H_2O$ molecule is easily removed from the adjacent C-4 hydroxyl group and C-9 hydroxyl group. These three types of "fugu toxins" are a little different in chemical properties, but are significantly different in bioactivity.

Therefore, we concluded that tetrodotoxin becomes unstable under the interaction of water molecule as a proton causes epimerization to form 4,9-dehydro-tetrodotoxin. In order to prevent epimerization of tetrodotoxin, the C-4 hydroxyl must not be epimerized. Thus, the best approach to improve the stability of tetrodotoxin is to reduce the C-4 hydroxyl. For this purpose, we synthesized 4-deoxy-tetrodotoxin with its structure shown below:

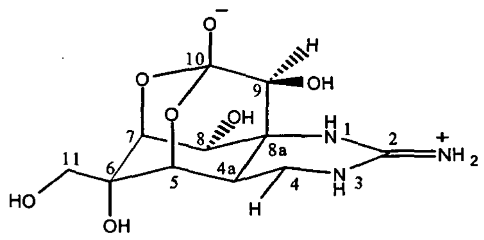

4-deoxy-Tetrodotoxin

4-Deoxy-tetrodotoxin is very stable as it did not change in a water solution after being boiled for 2 hours, as confirmed by HPLC analysis. However, its $LD_{50}$ is 3336.5 μg/kg and its toxicity is 330 times less than that of tetrodotoxin. Its analgesic effectiveness was examined with acetic acid induced writhing in mice, and the results indicated that its analgesic effectiveness was approximately 330 times lower than that of tetrodotoxin.

Based upon the above experimental results, we concluded that the C-4 hydroxyl group of the tetrodotoxin molecule is the key position for its bioactivity and plays a key role in bioactivity. Hence, it is critical to keep it at an equatorial position. A water molecule can change it to an axial position; therefore, the question (4) is raised: how can the conversion of tetrodotoxin be prevented? In order to improve the stability of tetrodotoxin when selecting the freeze-dried pharmaceutical formulation containing a trace amount of tetrodotoxin, it is necessary to find additives which are capable of locking the C-4 hydroxyl group at an equatorial position.

Therefore, the present inventors found solutions for the above problem.

As the stereochemical-position of the C-4 hydroxyl group of the tetrodotoxin molecule plays a key role in its bioactivity, the present inventors use this as the starting point to seek a pharmaceutically acceptable excipient(s) which do not cause the stereochemical-position of the C-4 hydroxyl group to change, and therefore prevent it from epimerizing. Firstly, the proton of the hydroxyl is prone to forming a hydrogen bond with an electronegative atom like an oxygen atom. There are six electrons in the outer shell of an oxygen atom, leaving two pairs of electrons unused after forming a chemical compound, thus resulting in a strong electronegativity which enables it to form hydrogen bonds with the proton of the C-4 hydroxyl group and the proton of the nitrogen atom. Forming of these hydrogen bonds results in a six-member ring, thereby "locking" the C-4 hydroxyl, i.e., the stereochemical-position of the C-4 hydroxyl is locked. Secondly, because the stereochemical structures of the two six-member rings in the tetrodotoxin molecule are chair forms, it was contemplated to find some compounds with a similar structure to tetrodotoxin and which can surround the tetrodotoxin molecule. Compounds meeting with these two requirements are those containing glucosidic bonds, such as disaccharides. Based upon above analysis and continuous studies, we have discovered that addition of a certain amount of disaccharide(s) like lactose, sucrose, cellobiose and maltose into the tetrodotoxin formulation then freeze-drying indeed improves its stability. After storing at room temperature for one year, the content of tetrodotoxin and related substances did not show significant change, and therefore the formulation meets the standard medicinal criteria. By way of example, lactose is used to explain the mechanism of preventing the C-4 hydroxyl group from epimerizing through the formation of a hydrogen bond between tetrodotoxin and a disaccharide.

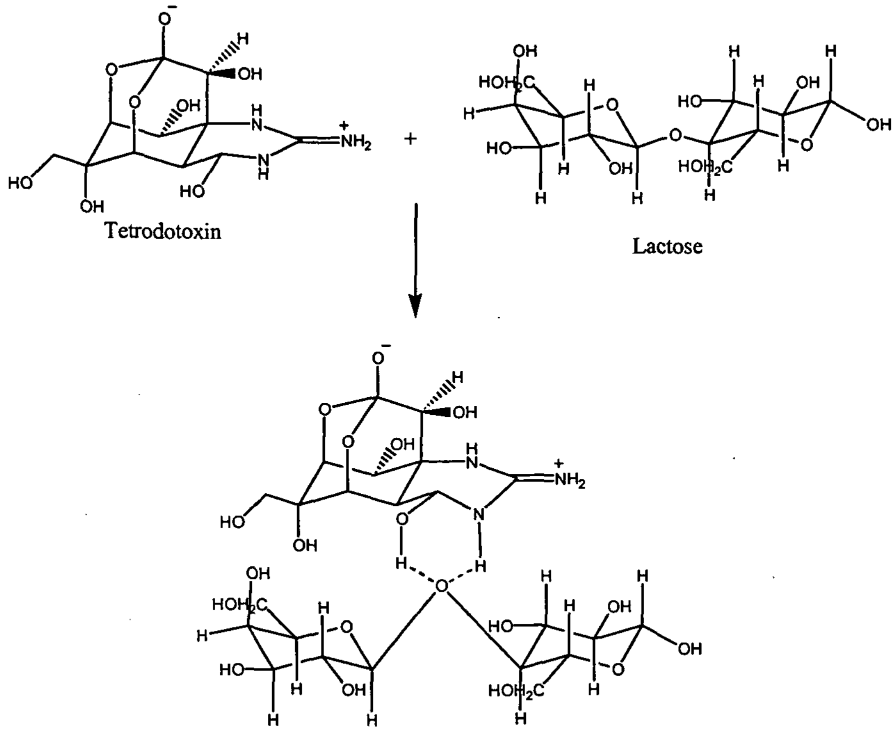


Locking C-4 hydroxyl through the formation of hydrogen bond between lactose and tetrodotoxin

Glucosidic bonds are also present in ficolls such as polyglucose and dextran, or derivatives thereof such as hydroxyethyl starch, hydroxypropyl cyclodextrin which are similar to tetrodotoxin in structure. Therefore, the study shows that ficoll is also able to stabilize tetrodotoxin. Those such as monosaccharides (glucose, fructose, and mannose) without glucosidic bonds cannot lock the stereochemical-position of the C-4 hydroxyl, and therefore fail to prevent tetrodotoxin from epimerizing. The reason could be parallel interaction between the dipole of the C-1 hydroxyl in β -isomer and the dipole of the epoxy in the monosaccharide, so as to lead to repulsion. Such dipole-dipole repulsion does not help the epoxy atom form a hydrogen bond in a six-member ring (Jingyan WANG et al., Biochemistry, Beijing Advanced Education Publishing, 2002,13).

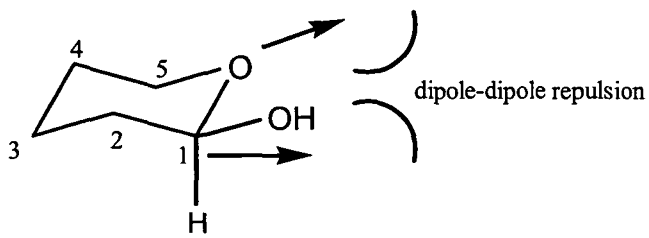


equatorial hydroxy (β isomer)

Parallel interaction between the dipole of C-1 hydroxyl of β -isomer and the dipole of the epoxy of the monosaccharide
The present invention can be carried out as follows:

To overcome the deficiencies in existing technology, i.e., the active ingredient, tetrodotoxin is not stable in an aqueous solution or in the form of freeze-dried citrate, the present inventors invented a stable freeze-dried pharmaceutical formulation of tetrodotoxin and a method of making the same. Said pharmaceutical formulation is a freeze-dried form and can be stored for a longer time at room temperature. Before using, the formulation can be regenerated by adding a pharmaceutically accepted aqueous solution, and then administered by injection.

The present invention provides a stable freeze-dried pharmaceutical formulation of tetrodotoxin comprising a

trace amount of bioactive ingredient, namely tetrodotoxin, stabilizer(s) and co-solvent(s), wherein said tetrodotoxin has an amount of 0.5-60 μg per dose, with remaining stabilizer(s) and co-solvent(s).

The tetrodotoxin includes tetrodotoxin or its analogs such as dehydro-tetrodotoxin, amino-tetrodotoxin, methoxy-tetrodotoxin and ethoxy-tetrodotoxin. The bioactive tetrodotoxin is extracted from the ovaries and livers of puffer fish, a marine animal; or other species such as amphibians, turbellaria, nemerteans, steroidea, sagitta, and gastropoda; or some bacteria such as vibrio alginolyticus. The extraction can be done by using methods disclosed in the prior art, such as U.S. Patent No. 6,552,191, Zhou, et al. The analogs thereof are obtained by modifying the structure of tetrodotoxin.

In this invention, tetrodotoxin is used as an active ingredient in a trace amount safe for use by injection in humans in dosages, ranging from 0.5 μg to 60 μg. A freeze-dried formulation cannot be made by using tetrodotoxin alone, therefore, a pharmaceutical excipient(s) should be added so as to increase the concentration of a solution before being freeze dried. The study found that tetrodotoxin during storage can only be prevented from epimerizing to 4-epi-tetrodotoxin and 4, 9-dehydro tetrodotoxin when one or more stabilizing substances are added. In this invention, such substances are lactose, sucrose, maltose, ficoll, including polyglucose, dextran; or an analogs thereof, including hydroxyethyl starch, hydroxypropyl cyclodextrin, in the range of 5-100 mg per dose.

The bioactive tetrodotoxin in this invention is hardly soluble in water, thus it is necessary to add a co-solvent to help tetrodotoxin dissolve and obtain required concentrations. Tetrodotoxin is an organic base chemically and therefore is soluble in acidic solutions. However, strong acids will decompose it and therefore non-volatile organic acids, preferably citric acid, tartaric acid, malic acid or lactobionic acid, form organic salts with tetrodotoxin to dissolve in water. Meanwhile, it is easy to control acidity. Experimental results suggest that the amount of co-solvent(s) is used in the formulation ranging from 0.00005 to 0.0005 mg and the pH value in the solution before freeze-drying is in the range of 3.0~6.0.

This invention provides a solid pharmaceutical formulation by freeze-drying an aqueous solution or a solution in a water soluble solvent of the bioactive tetrodotoxin and a disaccharide or polyglucose or an analog thereof (Reminton's Pharmaceutical Sciences, Seventeenth Edition, 1985, 1314). Said aqueous solution can be readily prepared by passing through a 0.22 μm membrane then a 10000 ultra filter and freeze drying to obtain a sterile and pyrogen-free freeze-dried powder, which is stable and the bioactive tetrodotoxin can be prevented from conversion for a long time.

In order to obtain a constant and suitable pH value for the freeze-dried powder and to prevent irritation to the local tissue or necrosis, a certain amount of co-solvent(s) is added to control the pH value in the range of 3.0~6.0. If the pH of the solution is lower than 3.0, it can be adjusted by adding diluted sodium (potassium) hydroxide, and if the pH value is between 3.0 and 6.0, it is not necessary to adjust it.

The present invention provides a method for preparing a freeze-dried pharmaceutical formulation of tetrodotoxin as follows: dissolving the co-solvents, stabilizers and pH-adjusting agents into water respectively; and dissolving a trace amount of tetrodotoxin in the certain amount of co-solvents, combining the above solutions; adding water for injection to a specified volume, testing whether the pH of the solution is in the range of 3.0~6.0; otherwise adjusting it with sodium (potassium) hydroxide or the corresponding sodium (potassium) salts of organic acids, removing bacteria by filtering and ultra-filtering, filling in vials, loosely placing stoppers, freeze drying, putting stoppers in place, and rolling covers on.

The aforementioned freeze-dried composition can be regenerated by adding an aqueous solution suitable for human use so as to obtain a clear liquid, sterile and pyrogen-free and ready for intramuscular or subcutaneous injection. Said aqueous solution may be sterile water or other aqueous solution suitable for injection, having a volume of 0.5-5 mL, preferably 1-2 mL.

The method for preparing said pharmaceutical formulation comprises the following steps:

1. dissolving a certain amount of a co-solvent into water for injection;
2. dissolving a pH-adjusting agent into water for injection;
3. dissolving a dissacchride or polyglucose or an analog thereof as a stabilizer into water for injection;
4. adding a trace amount of tetrodotoxin into a calculated amount of a solvent solution, stirring until dissolved;
5. adding 4 into 3, and adding water for injection to a specified volume, shaking well;
6. testing whether the pH of the solution is in the range of 3.0~6.0; otherwise, adjusting it with pH-adjusting solution;
7. sterilizing by filtering (e.g., with filtering system from Millipore) and ultra-filtering (e.g. with ultra-filtering system from Pull) to obtain a clear, sterile, and pyrogen-free solution;
8. aliquoting the resulting solution in 7 in vials with a specified amount in each vial, putting on covers loosely and placing into a freeze dryer; refrigerating until the surface temperature declines to under -40°C, then freezing further to under -50°C; switching on the vacuum pump; maintaining the pressure under 5 Pa, and allowing the temperature to rise without intervention to a specified level; allowing standing for 24 hours, and then escalating the temperature to 30°C for a period of 10 hours; closing the stoppers automatically; and
9. taking out the vials and rolling on covers.

Compared to the prior art, this invention has pertinent and significant features as follows:

It is difficult to store the tetrodotoxin injection in the refrigerator (4-8°C) or at room temperature. Epimerization readily occurs at room temperature to turn tetrodotoxin into 4-epi-tetrodotoxin and 4,9-dehydro-tetrodotoxin so that tetrodotoxin loses its pharmaceutical value. The inventors conducted extensive studies to find stabilizers which prevent tetrodotoxin from epimerizing to keep the trace amount of tetrodotoxin stable in the formulation at room temperature. More specifically:

1. stabilizers such as a disaccharide or polyglucose or an analog thereof are added in the formulation to effectively prevent tetrodotoxin from epimerizing during storage, i.e., prevent tetrodotoxin from turning into 4-epi-tetrodotoxin and 4, 9-dehydro-tetrodotoxin, and thereby to ensure the product quality;
2. freeze drying techniques are used to improve the stability of the product so that there is little water left in the formulation;
3. the combination of the above techniques can effectively solve the stability problem for a trace amount of tetrodotoxin in the pharmaceutical formulation;
4. it is not necessary to store the pharmaceutical formulation at 4-8°C, it can be stored at room temperature; therefore the storage and transportation costs can be reduced and it is convenient for clinical use; more importantly, this invention provides a safe and reliable product with a stable quality and which is storable at room temperature for more than one year.

## THE BEST MODE OF THE PRESENT INVENTION

### Example 1

Freeze-dried formulations comprising tetrodotoxin and a disaccharide (lactose, sucrose, maltose and cellobiose) were prepared in the amounts as specified in Table 7. Fructose (monosaccharide) was used as excipient in Formulation 1, whereas disaccharides were used as stabilizers (and also excipients) in Formulations 2, 3, 4 and 5. Citric acid (co-solvent) and the stabilizers were dissolved separately in water for injection; then tetrodotoxin was dissolved in the citric acid solution, followed by combining the stabilizer solution into the above solution and adding water for injection up to the specified volume. The resulting solution was stirred well, and its pH was adjusted to 4.0; then bacteria were removed by filtering and ultra-filtering. The resulting solution was filled in glass vials to the specified volume; then covers were put on loosely, and the vials were put in a freeze dryer. After the temperature of the vials was reduced to -40°C, the freeze chamber was switched on to further reduce the temperature to under -50°C. The vacuum pump was started to maintain the pressure under 5 Pa; then the temperature was allowed to rise without intervention to a specified level. After vials being allowed standing for 24 hours, the temperature was escalated naturally to 30°C for a period of at least 10 hours. The stoppers were closed tightly and covers were rolled on.

Table 7

| Formulation | 1 | 2 | 3 | 4 | 5 |
|---|---|---|---|---|---|
| Tetrodotoxin | 3 mg | 3 mg | 3 mg | 3 mg | 3 mg |
| Fructose | 3000 mg | - | - | - | - |
| Lactose | | 3000 mg | - | - | - |
| Sucrose | - | - | 3000 mg | - | - |
| Maltose | - | - | - | 3000 mg | - |
| Cellobiose | - | - | - | - | 3000 mg |
| Citric acid | 0.012 mg | 0.012 mg | 0.012 mg | 0.012 mg | 0.012 mg |
| Water for injection | add to 100ml | | | | |

Stability tests were conducted for the above freeze-dried formulations and the tetrodotoxin injection (liquid form) at 40°C at the same time. Samples were taken on day 1, 3, 5, and 10. The content of tetrodotoxin and related substances were measured by HPLC, and compared with day 0. Results are shown in Table 8.

Table 8 Results of Stability Studies at 40°C for Tetrodotoxin Injection (liquid form) and Various Freeze-Dried Tetrodotoxin Formulations

| Formulations | 1 | | 2 | | 3 | | 4 | | 5 | | 6* | |
|---|---|---|---|---|---|---|---|---|---|---|---|---|
| Appearance | No crystal or powder was formed. The appearance did not meet the criteria. | | Slack white cake-shaped solid. No obvious change was found after standing for 10 days at 40°C. The appearance met the criteria. | | Slack white cake-shaped solid. No obvious change was found after standing for 10 days at 40°C. The appearance met the criteria. | | Slack white cake-shaped solid. No obvious change was found after standing for 10 days at 40°C. The appearance met the criteria. | | Slack white cake-shaped solid. No obvious change was found after standing for 10 days at 40°C. The appearance met the criteria. | | Colorless, clear liquid. No obvious change was found after standing for 10 days at 40°C . | |
| Content and related substances | Content (%) | Related Substances (%) | Content (%) | Related Substances (%) | Content (%) | Related Substances (%) | Content (%) | Related Substances (%) | Content (%) | Related Substances (%) | Content (%) | Related Substances (%) |
| Limit | 90-110 | <main peak area of control solution | 90-110 | <main peak area of control solution | 90-110 | <main peak area of control solution | 90-110 | <main peak area of control solution | 90-110 | <main peak area of control solution | 90-110 | <main peak area of control solution |
| Day 0 | 100 | <main peak area of control solution | 100 | <main peak area of control solution | 100 | <main peak area of control solution | 100 | <main peak area of control solution | 100 | <main peak area of control solution | 100 | <main peak area of control solution |
| Day 1 | 99.7 | <main peak area of control solution | 100 | <main peak area of control solution | 99.46 | <main peak area of control solution | 99.55 | <main peak area of control solution | 99.47 | <main peak area of control solution | 98.53 | <main peak area of control solution |

| | | | | | | | | | | | | |
|---|---|---|---|---|---|---|---|---|---|---|---|---|
| Day 3 | 99.06 | <main peak area of control solution | 99.72 | <main peak area of control solution | 99.24 | <main peak area of control solution | 99.21 | <main peak area of control solution | 99.61 | <main peak area of control solution | 96.66 | >main peak area of control solution |
| Day 5 | 97.54 | >main peak area of control solution | 99.73 | <main peak area of control solution | 99.77 | <main peak area of control solution | 99.69 | <main peak area of control solution | 99.72 | <main peak area of control solution | 92.73 | >main peak area of control solution |
| Day 10 | 95.99 | >main peak area of control solution | 99.97 | <main peak area of control solution | 99.56 | <main peak area of control solution | 99.18 | <main peak area of control solution | 99.75 | <main peak area of control solution | 90.06 | >main peak area of control solution |

Notes: 1. 6* is a tetrodotoxin injection (liquid form)

2. The criteria are not met if the content of tetrodotoxin is less than 90% or related substances' content is greater than the main peak of the control solution; then the product cannot be used as a medicine.

Fructose was used as excipient in Formulation 1, of which the appearance did not meet the criteria. At the 40°C test, the content of tetrodotoxin in this formulation declined gradually from 100% on day 0 to 95.99% on day 10, or a decrease of 4.01%. On the other hand, the content area of related substances exceeded the major peak area of the control solution, not meeting the criteria. Therefore, fructose is unable to prevent tetrodotoxin from epimerizing, and is not useful to preserve the stability of tetrodotoxin.

Disaccharides such as lactose, sucrose, maltose and cellobiose, were used as stabilizers in formulations 2, 3, 4 and 5, respectively. At 40°C standing for 10 days, these formulations did not have significant changes in the content of tetrodotoxin or related substances. The content of tetrodotoxin was 99.97%, 99.56%, 99.18%, and 99.75%, respectively, while the content areas of related substances were all smaller than the major area of the control solution. These results indicate that these formulations have greatly improved stability and meet the criteria for medicines, and disaccharides are capable of preventing tetrodotoxin from epimerizing and thus achieving the goals of the invention. Formulation 6 is a liquid form of the tetrodotoxin injection, which under the same test conditions had a gradually declining content of tetrodotoxin from 100% on day 0 to 90.06% on day 10, or a decrease of 9.94%, whereas the content area of related substances exceeded the major peak area of the control solution starting from day 3, not meeting the criteria. Therefore, these results suggest that freeze-dried tetrodotoxin formulations with disaccharides as stabilizers have a higher stability than the liquid form of the tetrodotoxin injection.

A long term stability study was conducted at room temperature on the above freeze-dried tetrodotoxin formulations and the liquid form of the tetrodotoxin injection, with samples taken in month 1, 2, 3, 6, 9 and 12. The content of tetrodotoxin and related substances were measured by HPLC, and compared with day 0. Results were presented in Table 9.

Table 9 Results of Stability Studies at Room Temperature for Tetrodotoxin Injection (liquid form) and Various Freeze-Dried Tetrodotoxin Formulations

| Formulations | 1 | | 2 | | 3 | | 4 | | 5 | | 6* | |
|---|---|---|---|---|---|---|---|---|---|---|---|---|
| Appearance | No crystal or powder was formed. The appearance did not meet the criteria. | | Slack white cake-shaped solid. No obvious change was found after standing at room temperature. The appearance met the criteria. | | Slack white cake-shaped solid. No obvious change was found after standing for at room temperature. The appearance met the criteria. | | Slack white cake-shaped solid. No obvious change was found after standing for at room temperature. The appearance met the criteria. | | Slack white cake-shaped solid. No obvious change was found after standing for at room temperature. The appearance met the criteria. | | Colorless, clear liquid. No obvious change was found after standing at room temperature. | |
| Content and related substances | Content (%) | Related Substances (%) | Content (%) | Related Substances (%) | Content (%) | Related Substances (%) | Content (%) | Related Substances (%) | Content (%) | Related Substances (%) | Content (%) | Related Substances (%) |
| Limit | 90-110 | <main peak area of control solution | 90-110 | <main peak area of control solution | 90-110 | <main peak area of control solution | 90-110 | <main peak area of control solution | 90-110 | <main peak area of control solution | 90-110 | <main peak area of control solution |
| Month 0 | 100 | <main peak area of control solution | 100 | <main peak area of control solution | 100 | <main peak area of control solution | 100 | <main peak area of control solution | 100 | <main peak area of control solution | 100 | <main peak area of control solution |
| Month 1 | 97.53 | <main peak area of control solution | 100.07 | <main peak area of control solution | 99.78 | <main peak area of control solution | 100.48 | <main peak area of control solution | 99.97 | <main peak area of control solution | 95.34 | <main peak area of control solution |
| Month 2 | 94.16 | >main peak area of control solution | 100.08 | <main peak area of control solution | 99.5 | <main peak area of control solution | 100.07 | <main peak area of control solution | 99.86 | <main peak area of control solution | 92.58 | >main peak area of control solution |

| | | | | | | | | | | | | |
|---|---|---|---|---|---|---|---|---|---|---|---|---|
| Month 3 | 92.09 | >main peak area of control solution | 100.1 | <main peak area of control solution | 100.79 | <main peak area of control solution | 99.83 | <main peak area of control solution | 100.68 | <main peak area of control solution | 89.07 | >main peak area of control solution |
| Month 6 | 90.52 | >main peak area of control solution | 100.13 | <main peak area of control solution | 99.53 | <main peak area of control solution | 100.3 | <main peak area of control solution | 99.29 | <main peak area of control solution | 82.25 | >main peak area of control solution |
| Month 9 | 86.27 | >main peak area of control solution | 100.04 | <main peak area of control solution | 100.2 | <main peak area of control solution | 99.65 | <main peak area of control solution | 99.32 | <main peak area of control solution | 77.06 | >main peak area of control solution |
| Month 12 | 83.19 | >main peak area of control solution | 101.92 | <main peak area of control solution | 99.89 | <main peak area of control solution | 99.87 | <main peak area of control solution | 99.47 | <main peak area of control solution | 73.38 | >main peak area of control solution |

Notes: 1. 6* is a tetrodotoxin injection (liquid form)

2. The criteria are not met if the content of tetrodotoxin is less than 90% or related substances' content is greater than the main peak of the control solution; then the product cannot be used as a medicine.

Fructose was used as excipient in Formulation 1, of which the appearance did not meet the criteria. During the storage period, the tetrodotoxin content declined from 100% in month 0 to 83.19% in month 12, or a drop of 16.81%. In month 2, the content of related substances exceeded the major peak area of the control solution, not meeting the criteria and not qualified for medical use. Disaccharides such as lactose, sucrose, maltose and cellobiose were used as stabilizers in formulations 2, 3, 4 and 5, respectively. At room temperature standing for 12 months, these formulations did not have significant changes in appearance, the content of tetrodotoxin or related substances. The content of tetrodotoxin was 101.92%, 99.89%, 99.87%, and 99.47%, respectively, while the content areas of related substances were all smaller than the major area of the control solution, meeting the criteria for medical use. Formulation 6 is the liquid form of the tetrodotoxin injection, which under the same storage conditions had a content of tetrodotoxin declining from 100% in month 0 to 73.38% in month 12, a drop of 26.62%. After month 2, its content area of related substances had exceeded the major peak area of the control solution, not meeting the criteria and indicating a poor stability. Therefore, disaccharides are capable of protecting a trace amount of tetrodotoxin very well so that the content of tetrodotoxin and related substances meet the requirements for clinical use even after 12 months of storage at room temperature. Hence, the stability of tetrodotoxin formulations is maintained.

**Example 2**

The method for preparing a freeze-dried formulation containing 30μg bioactive tetrodotoxin and 30mg dextran is as follows:
The method described in Example 1 was followed to obtain a slack white cake-shaped solid by freezing dry a solution of 0.003% tetrodotoxin and 3% dextran, pH 3.0. Then its stability at 40°C was studied with samples taken on day 1, 3, 5, 10; the content of tetrodotoxin and related substances were measured by HPLC, and compared to the results of day 0, as shown in Table 10.

Table 10

| Appearance | Slack white cake-shaped solid. No obvious change was observed after standing for 10 days at 40°C, thus the appearance met with the criteria. | |
|---|---|---|
| Content | Tetrodotoxin (%) | Related substances (%) |
| Limit | 90-110 | <main peak area of control solution |
| Day 0<br>Day 1<br>Day 5<br>Day 10 | 100<br>99.46<br>98.63<br>98.13 | <main peak area of control solution<br><main peak area of control solution<br><main peak area of control solution<br><main peak area of control solution |
| Conclusion | medicinal criteria met | |

The results show that dextran stabilizes tetrodotoxin in this formulation as its chemical structure is similar to disaccharides. At the high temperature of 40°C, the appearance, the content of tetrodotoxin and related substances meet medicinal standards.

**Example 3**

The method for preparing a freeze-dried powder formulation containing 60μg bioactive tetrodotoxin and 5 mg lactose (or sucrose, maltose, cellobiose):

The method described in Example 1 was followed to obtain a slack white cake-shaped solid by freezing dry a solution of 0.006% tetrodotoxin and 0.5% lactose, pH 4.0. The above solid is dissolved into sterile water for injection or pharmaceutically acceptable aqueous solution to get a sterile and pyrogen-free clear solution which can be directly used for intramuscular or subcutaneous injection.

**Example 4**

The method for preparing a freeze-dried powder formulation containing 0.5 μg bioactive tetrodotoxin and 100 mg lactose (or sucrose, maltose, cellobiose) is as follows:

The method described in Example 1 was followed to obtain a slack white cake-shaped solid by freezing dry a solution

of 0.00005% tetrodotoxin and 10% lactose, pH 6.0. After being dissolved in an aqueous solution, the resultant solution can be directly used for intramuscular or subcutaneous injection.

### Example 5

The method for preparing a freeze-dried powder formulation containing 5 μg bioactive tetrodotoxin, 15 mg lactose and 15 mg sucrose (or maltose, cellobiose) is as follows:

The method described in Example 1 was followed to obtain a slack white cake-shaped solid by freezing dry a solution of 0.0005% tetrodotoxin, 1.5% lactose and 1.5% sucrose, pH 4.5. The above solid is dissolved in sterile water for injection or a pharmaceutically acceptable aqueous solution to get a sterile and pyrogen-free clear solution which can be directly used for intramuscular or subcutaneous injection.

### Example 6

The method for preparing a freeze-dried powder formulation containing 20 μg bioactive tetrodotoxin, 15 mg lactose (or sucrose, maltose, cellobiose) and 15 mg mannitol is as follows:

The method described in Example 1 was followed to obtain a slack white cake-shaped solid by freezing dry a solution of 0.002% tetrodotoxin, 1.5% lactose and 1.5% mannitol, pH 5.5. The above solid is dissolved in sterile water for injection or a pharmaceutically acceptable aqueous solution to get a sterile and pyrogen-free clear solution which can be directly used for intramuscular or subcutaneous injection.
The samples obtained through this method had a tetrodotoxin content of 99.65% after being stored for one year at room temperature, and its content of related substances was smaller than the major peak area of the control solution, meeting the requirements for clinical use, whereas using mannitol alone failed to achieve this objective. Therefore, this demonstrates that using lactose, sucrose, maltose, or cellobiose in the freeze-dried formulation can significantly maintain the stability of the formulation of tetrodotoxin.

### Industrial Applicability

The freeze-dried formulation of the present invention can be used as a pharmaceutical formulation.

## Claims

1. A stable freeze-dried pharmaceutical formulation, comprising tetrodotoxin or an analog thereof as an active ingredient, in **characterized that** said tetrodotoxin or the analog thereof is in an amount of 0.5-60 μg per dose, said the formulation also comprises a compound capable of reducing the activity of C-4 hydroxyl of tetrodotoxin molecule or the analog thereof.

2. The formulation of claim 1, wherein said compound capable of reducing the activity of C-4 hydroxyl of tetrodotoxin molecule or the analog thereof is the compound containing a glucosidic bond.

3. The formulation of claim 2, wherein said compound containing a glucosidic bond is selected from the group consisting of a disaccharide, a ficoll, or a derivative thereof or one of the mixtures thereof.

4. The formulation of any one of claims 1 to 3, wherein tetrodotoxin or the analog thereof is selected from the group consisting of tetrodotoxin, anhydrotetrodotoxin, amino-tetrodotoxin, methoxytetrodotoxin and ethoxytetrodotoxin.

5. The formulation of claim 3, wherein said compound containing a glucosidic bond is present in an amount of 5-100 mg per dose.

6. The formulation of claim 3, wherein said disaccharide is selected from the group consisting of lactose, sucrose, maltose and cellobiose, and said ficoll is selected from the group consisting of polyglucose and dextran, and said derivative is selected from the group consisting of hydroxyethyl starch and hydroxypropyl cyclodextrin.

7. The formulation of claim 1, said formulation also comprises a co-solvent selected from the group consisting of citric acid, tartaric acid, malic acid and lactobionic acid, and said co-solvent is present in an amount that makes the pH

of the solution to 3.0~6.0 before being freeze-dried.

**8.** The formulation of claim 7, wherein said co-solvent is present in an amount of 0.00005-0.0005 milligram per dose.

**9.** The formulation of claim 1, said active ingredient is tetrodotoxin.

**10.** The formulation of claim 1, said formulation also comprises a pharmaceutically acceptable excipient(s).

# INTERNATIONAL SEARCH REPORT

International application No.
PCT/CN2004/000736

A. CLASSIFICATION OF SUBJECT MATTER

IPC7 A61K31/529; A61K9/19; A61P25/04

According to International Patent Classification (IPC) or to both national classification and IPC

B. FIELDS SEARCHED

Minimum documentation searched (classification system followed by classification symbols)

IPC7 A61K; A61P

Documentation searched other than minimum documentation to the extent that such documents are included in the fields searched

CHINA PATENT DOCUMENTS, CHINESE MAGAZINES

Electronic data base consulted during the international search (name of data base and, where practicable, search terms used)

WPI, EPODOC, PAJ, CA, MEDLINE, EMBASE, CPRS, CFPASS, CNKI tetrodotoxin/TTX freeze drying/ lyophilization

C. DOCUMENTS CONSIDERED TO BE RELEVANT

| Category* | Citation of document, with indication, where appropriate, of the relevant passages | Relevant to claim No. |
|---|---|---|
| A | WO95/24903A1(PAN, Xinfu et al.)21. SEP 1995 (21.09.1995), see the whole document | 1-10 |
| A | CN1145225A(WANG, Weiguo )19. MAR 1997 (19.03.1997), see the whole document | 1-10 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 06. SEP 2004 (06.09.2004) | 14 · OCT 2004 (14 · 10 · 2004) |

| Name and mailing address of the ISA/CN | Authorized officer |
|---|---|
| 6 Xitucheng Rd., Jimen Bridge, Haidian District, 100088 Beijing, China | GUO Ting |
| Facsimile No. (86-10)62019451 | Telephone No. (86-10)62085229 |

印 郭 婷

Form PCT/ISA /210 (second sheet) (January 2004)

**INTERNATIONAL SEARCH REPORT**
Information on patent family members

International application No.
PCT/CN2004/000736

| Patent document Cited in search report | Publication date | Patent family member(s) | Publication date |
|---|---|---|---|
| WO9524903A1 | 21.09.95 | DE69529156E | 23.01.2003 |
| | | AU1888195A | 03.10.1995 |
| | | EP0750909A1 | 02.01.1997 |
| | | JP9510221T | 14.10.1997 |
| | | US5846975A | 08.12.1998 |
| | | RU2168331C2 | 10.06.2001 |
| | | EP0750909B1 | 11.12.2002 |

Form PCT/ISA /210 (patent family annex) (January 2004)